# EUROPÄISCHE PATENTSCHRIFT

(11) **EP 0 915 713 B1**
(45) Veröffentlichungstag und Bekanntmachung des Hinweises auf die Patenterteilung: **22.08.2001**
(21) Anmeldenummer: 97931630.4
(22) Anmeldetag: 20.06.1997
(51) Int. Cl.: A61L 9/16, A61L 9/20

(54) **VORRICHTUNG ZUM REINSTFILTERN UND DESINFIZIEREN VON LUFT**
DEVICE FOR SHEER FILTERING AND DESINFECTION OF AIR
DISPOSITIF DE FILTRAGE ULTRA-PUR ET DE DESINFECTION DE L'AIR

(30) Priorität: 18.12.1996 DE 19652688
(43) Veröffentlichungstag der Anmeldung: 19.05.1999
(73) Patentinhaber: Tillmanns, Kurt, 56072 Koblenz (DE)
(72) Erfinder: Tillmanns, Kurt, 56072 Koblenz (DE)
(74) Vertreter: Grünecker, Kinkeldey, Stockmair & Schwanhäusser Anwaltssozietät
(86) Internationale Anmeldenummer: DE9701283
(87) Internationale Veröffentlichungsnummer: WO9826810

(56) Entgegenhaltungen:
- EP-A- 0 461 310
- WO-A-95/17634
- WO-A-95/35123
- DE-U- 7 221 461
- DE-U- 9 313 409
- DE-U- 29 622 001
- US-A- 2 500 007

## Beschreibung

Die Erfindung betrifft eine Vorrichtung zum Reinstfiltem und Desinfizieren von Luft.

Aus der WO 95/17634 ist ein Verfahren und eine Vorrichtung, in Form eines Einbaugeräts, zur Bestrahlung und zum Filtern von Raumluft bekannt, wobei eine Vorfilterstufe, eine UV-Bestrahlungsstufe und eine Feinstfilterstufe vorgesehen sind.

Die DE 72 21 461 U zeigt ebenfalls ein derartiges Luftfiltergerät mit drei Stufen, das jedoch als Standgerät ausgebildet ist.

In der Luft befinden sich vielerlei Verunreinigungen, die im industriellen Bereich großen Schaden anrichten können. Zum Beispiel können in Archiven, die auf Sicherheitsfilmen abgespeicherten Zeichnungen verloren gehen, wenn die Sicherheitsfilme durch Mikroorganismen befallen werden.

Verunreinigungen in der Atemluft können ebenfalls zu erheblichen Schäden führen. So können durch Bakterien. Viren und Schimmelpilze Mitarbeiter erkranken, deren Lohnfortzahlungen bedeutend zu Buche schlagen.

Weiterhin führen Pollen häufig zu Schleimhautreizungen, die erhebliches Unwohlsein zur Folge hat.

Insbesondere aber auch Menschen mit verminderter Widerstandskraft, wie solchen, die an Hepatitis C oder an Immunschwächen erkrankt sind, des weiteren Dialysepatienten, Allergiker, Asthmatiker und solchen, die an Neurodermatitis erkrankt sind, können durch die Schwebstoffe oder Mikroorganismen in dem Heilungsprozeß beeinträchtigt werden.

Allein in der Bundesrepublik werden etwa 30 Millionen Menschen durch Verunreinigungen der Atemluft in ihrem gesundheitlichen Wohlbefinden beeinträchtigt.

Damit Allergiker sich auf die oftmals erheblichen gesundheitlichen Störungen vorbereiten können, werden zu gegebenen Zeiten zu dem Wetterbericht auch Hinweise gegeben auf die Art der Blütenpollen, mit denen zu rechnen ist.

Auch in Krankenzimmem und auf Säuglingsstationen können Bakterien, Viren, Schimmelpilze die Gesundheit erheblich schädigen,

Bei ruhigem Liegen z.B. im Krankenzimmer atmet ein Erwachsener etwa 5 Liter Luft in einer Minute ein.

Bei ruhigem Stehen werden etwa 8 Liter Luft pro Minute geatmet.
Bei langsamem Gehen steigt die Luftmenge auf 17 Liter an.
Bei sportlicher Betätigung, z.B. Wettrudern, werden von einem Erwachsenen bis zu 140 Liter in einer Minute geatmet.

im Wohnbereich kann von einem Luftverbrauch von etwa 12 Liter pro Minute ausgegangen werden.

In Wartezimmern, Büros, Gaststätten, Flugzeugen ist häufig ein Luftaustausch nicht vorgesehen, es wird teilweise nur eine kleine Menge Frischluft der Klimaanlage zugeführt, so daß alle Viren, Bakterien überall hingetragen werden.

Die Verunreinigungen der Atemluft lassen sich in drei Gruppen zusammenfassen:
1. Verunreinigungen durch Schwebstoffe
   a) Hausstaub
   b) Milben
   c) Hautschuppen
   d) Ruß
   e) Pollen
   f) andere organische Verschmutzungen
2. Verunreinigungen durch Mikroorganismen
   g) Bakterien
   h) Viren
   i) Schimmelpilze
3. Verunreinigungen in Form von Gasen
   j) Formaldehyd
   k) Kohlenmonoxyd
   l) giftige Dämpfe
   m) unangenehme Gerüche z.B. Zigarettenrauch

Aufgabe der Erfindung ist es, Luft, insbesondere Atemluft reinstzufiltern und zu desinfizieren.

Gelöst wird die Aufgabe gemäß der Erfindung durch eine Vorrichtung mit den Merkmalen gemäß Anspruch 1.

An drei Ausführungen wirc die Erfindung näher erläutert.

Es zeigen:
Figur 1 eine Vorderansicht einer ersten Ausführung der erfindungs-gemäßen Reinstfilter- und Desinfiziervorrichtung
Figur 2 einen Vertilcalschnitt
Figur 3 den Vertikalschnitt einer zweiten Ausführung
Figur 4 die Seitenansicht der zweiten Ausführung
Figur 5 den Vertikalschnitt einer dritten Ausführung
Figur 6 die teilweise geschnittene Seitenansicht der dritten Ausführung

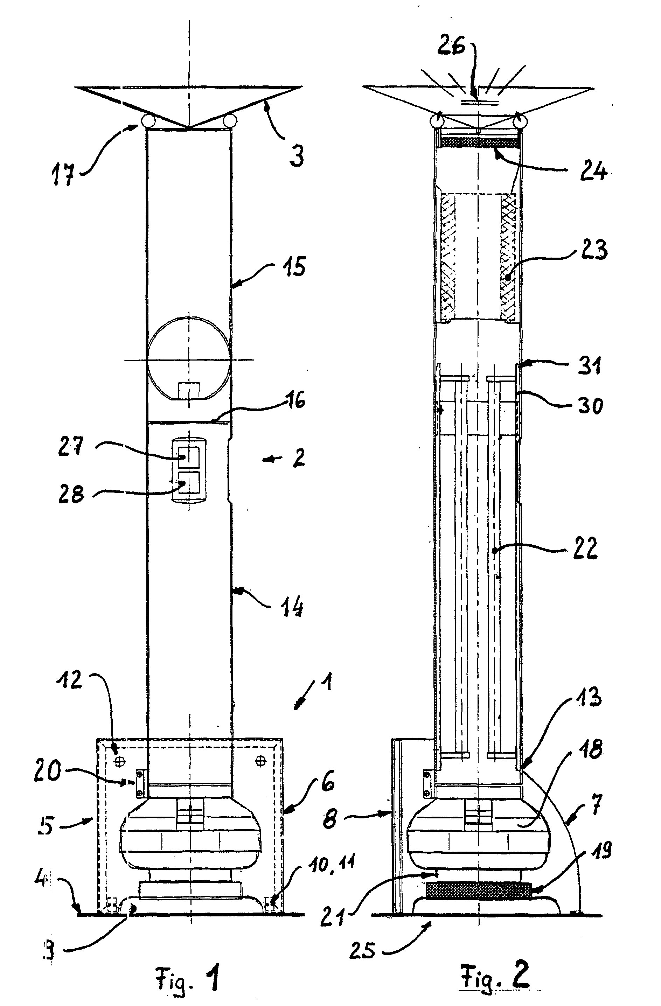

In den Figuren 1 und 2 ist eine erste Ausführung einer erfindungsgemäßen Vorrichtung dargestellt. Die Vorrichtung läßt sich äußerlich in drei Bereiche einteilen:
einem unteren Bereich, dem Gehäuse 1, einem mittleren Bereich,
dem Rohr 2 und einem oberen Bereich, dem Ausströmkegel 3.

Das Blechgehäuse 1 besteht aus einer ebenen Bodenplatte 4, den ebenen Seitenwänden 5, 6, einer gebogenen Vorderwand 7 und einer abnehmbaren Rückwand 8.
Die Seitenwände 5, 6 und die Vorderwand 7 werden auf der Bodenplatte 4 befestigt. Vorzugsweise wird die Befestigung durch eine Schweißverbindung hergestellt Des weiteren werden die Seitenwände 5, 6 und die Vorderwand 7 an den aneinanderliegenden Kanten ebenfalls verschweißt.

Die Seitenwände 5, 6 und die Vorderwand 7 weisen in ihrem der Bodenplatte 4 zugekehiten Bereich Öffnungen 9 auf. Diese Öffnungen 9 dienen der Ansaugung der Bodenluft.

Die Rückwand 8 ist leicht abnehmbar befestigt. Im unteren Bereich wird sie in zwei Öffnungen 10 mittels Blechzungen 11 gehalten, im oberen Bereich mittels zweier Schrauben 12.

Die Vorderwand 7 weist im oberen Bereich eine Öffnung 13 auf, durch die das Rohr 2 in das Blechgehäuse 1 eingeführt wird. Das Rohr 2 wird mit dem Rand der Öffnung 13 verschweißt.

Das Rohr 2 besteht aus einem unteren Rohrteil 14 und einem oberen Rohrteil 15. Beide Rohrteile 14/15 werden zusammengesteckt. Mit 16 ist die Trennebene der Rohrteile 14, 15 gekennzeichnet.

Der untere mit dem Gehäuse 1 verschweißte Rohrteil 14 ist bis zur Trennebene 16 von gleichbleibendem Durchmesser. Ab der Trennebene 16 ist der Außendurchmesser vermindert. Das Rohrteil 14 ragt dann noch ein wenig über die Trennebene mit vermindertem Außendurchmesser hinaus.
Der obere Rohrteil 15 weist im Bereich der Trennebene 16 verminderten Innendurchmesser auf, derart, daß beide Rohrteile gut zusammengesteckt werden können. Statt die Rohre in Ihrem Durchmesser zu bearbeiten, kann auch ein weiteres Rohrstück, dessen Außendurchmesser annähernd dem Innendurchmesser der Rohrteile 14, 15 entspricht, in das Rohrteil 14 eingeschweißt werden. Der aus dem Rohrteil 14 herausragende Teil übemimmt dann die Aufnahme des Rohrteiles 15.

Der obere Bereich, der Ausströmkegel 3, wird über mehrere gleichmäßig auf dem Rand de Rohres 2 verteilten Kugeln 17 mit dem Rohrteil 15 verbunden.

### Zum Aufbau im Inneren der Verbindung:

In dem Gehäuse 1 befindet sich ein Ventilator 18 und ein Feinstaubfilter 19. Der Ventilator 18, vorzugsweise ein Rohrventilator, wird mittels einer mit Isolierstoff ausgefütterten Schlauchschelle 20 an dem in das Gehäuse 1 ragenden Ende des Rohrteiles 14 befestigt. Diese Art der Befestigung dient dazu, die Schwingungen des Ventilators möglichst wenig auf das Rohr 2 zu übertragen.

Auf der Bodenplatte 4 zugewandten Seite des Ventilators 18 ist ein Gehäuse 21 befestigt, das den Feinstaubfilter 19 aufnimmt.

Das Gehäuse 2 weist einen rohrförmigen Abschnitt auf, der mit dem rohrförmigen Ausgangs Stutzen des Ventilators 18 schallisolierend verbunden ist. An den rohrförmigen Abschnitt schließt sich eine ebene Platte an, die an zwei gegenüberliegenden Seiten U-Schienen aufweist. Der Feinstaubfilter 19 wird in diese U-Schienen eingeschoben. Mittels Flügelschrauben wire der Feinstaubfilter gegen abdichtende Gummileisten an die ebene Platte angedrückt.
Der Feinstaubfilter 19 dient als Vorfilter. Er gehört zur Klasse F6.

In dem Rchr 2 befinden sich die UV-C Lampen 22, der Hauptfilter 23 sowie ein Aktivkohlefilter 24.
Der Hauptfilter 23 gehört zur Klasse EU 13/14 S der europäischen Norm. Diese Klasse beinhaltet die reinsten Filter, die es zur Zeit gibt. Sie filtern Körper der Größe ¹/_{300000 mm} heraus.

Die UV-C Lampen 22 sind hauptsächlich im unteren Bereich des Rohres 2 angeordnet, insbesondere sind die Lampensockel im unteren Rohrteil 14 befestigt.

Der Hauptfilter 23 kann mittels Schraubverschlusses in der oberen Rohrhälfte befestigt werden.

### Wirkungsweise:

Die von dem Ventilator 18 durch die Öffnungen 9 angesaugte Bodenluft 25 durchströmt den Feinstaubfilter 19. In diesem Feinstaubfilter werden die Verunreinigungen der ersten Gruppe, der Schwebstoffe, herausgefiltert.
Durch das Herausfiltem der Schwebstoffe können sich die UV-C Lampen nicht zusetzen, ihre Strahlungsfähigkeit bleibt lange erhalten, ca. 8000 Stunden.

Im nächsten Schritt wird die Atemluft durch das Rohr an den UV-C Lampen vorbeigeführt. Durch das UV-C Licht werden die meisten Bakterien, Viren und Mikroorganismen abgetötet.

Im dritten Schritt wird die vorgefilterte und nun desinfizierte Atemluft durch den Hauptfilter - einen Absolutfilter - geleitet.
Hier werden die abgetöteten Bakterien, Viren und Mikroorganismen herausgefiltert.

Im letzten Schritt wird die Atemluft noch durch einen agglomerierten Aktivkohlefilter geführt. Dieser soll die Atemluft von giftigen Dämpfen und unangenehmen Gerüchen reinigen.

Die nun so gefilterte Atemluft strömt am oberen Ende des Rohres durch den Freiraum zwischen den Kugeln 17 gegen den Ausströmkegel 3. Durch den Ausströmkegel wird die Atemluft gleichmäßig verteilt.

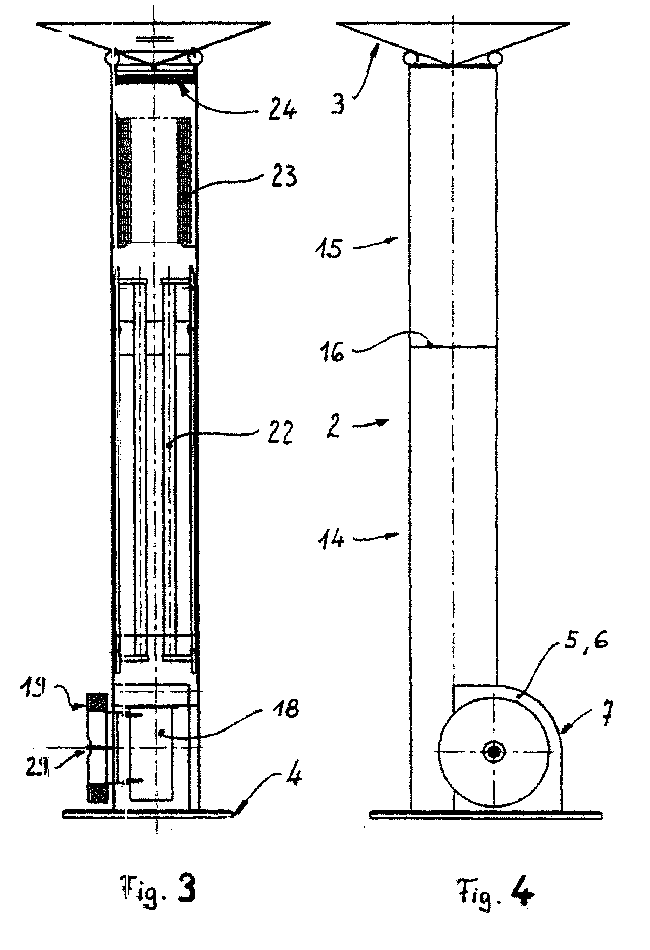

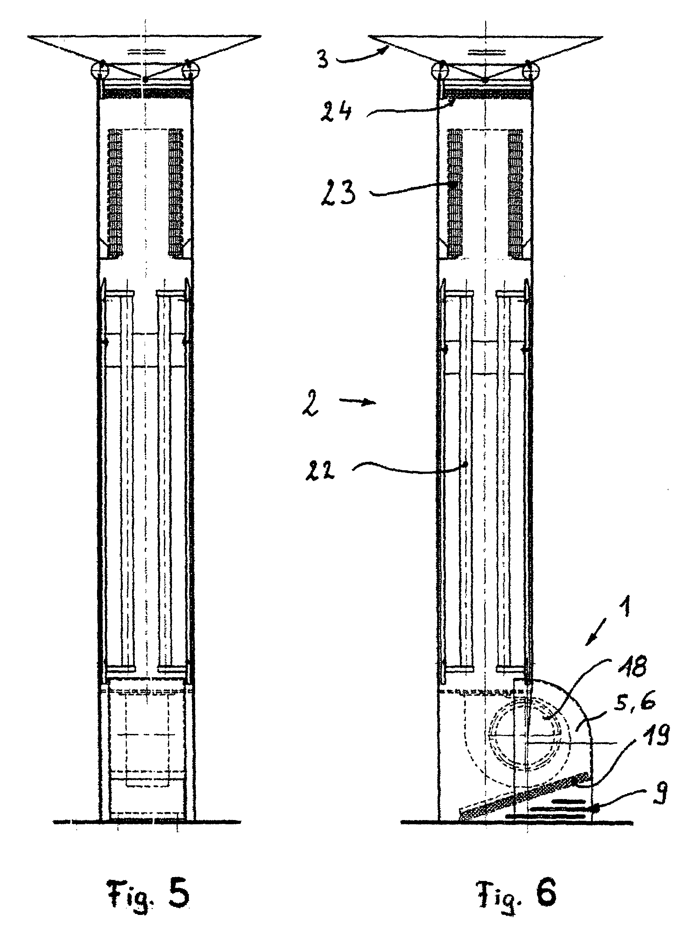

Die Figuren 3 und 4 zeigen das zweite Ausführungsbeispiel. Es unterscheidet sich von dem ersten durch die Anordnung des Feinstaubfilters 19 und des Ventilators 18.

Während bei der ersten Ausführung die Rückwand 8 abzunehmen war, um den Feinstaubfilter 19 auszutauschen, ist bei der zweiten Ausführung der Feinstaubfilter 19 außerhalb des Gehäuses 1 angeordnet.

Es genügt, die Schraube 29 zu lösen, um den Feinstaubfilter abnehmen zu können. Der Ventilator 18 war in der Ausführung gemäß der Figur 2 ein Axialgebläse, in der zweiten Ausführung wird ein Radialgebläse verwendet.

Desweiteren werden die Seitenwände 5 und 6 direkt mit dem unteren Rohrteil 14 verschweißt, derart, daß diese die Rückwand 8 bildet.

Die Wirkungsweise entspricht dem ersten Ausführungsbeispiel, außer, daß die Luft nun direkt zu dem Feinstaubfilter fließt und nicht mehr durch die Öffnungen 9.

Die Figuren 5 und 6 zeigen das dritte Ausführungsbeispiel.
Bei diesem wird wie im zweiten Ausführungsbeispiel ein Radialgebläse 18 verwendet. Der Feinststaubfilter 19 befindet sich innerhalb des Gehäuses 1. Die Seiten weisen statt einer großen Öffnung mehrere schmale Schlitze 9 auf. Die Wirkungsweise entspricht dem ersten Ausführungsbeispiel.

### Weitere Vorteile:

Durch den großen Abstand, zwischen 1,5 m und 2,5 m, insbesondere etwa 1,80m, von Ansaugöffnung 9 der Bodenluft 25 und Ausströmkegel 3 wird sichergestellt, dass die ausströmende Luft sich erst im Raum verteilt, sich mit der anderen Raumluft mischt, bevor sie wieder angesaugt wird. Es wird zuverlässig vermieden, dass sich ein kurzer Kreislauf bildet, derart, dass die ausströmende Luft direkt wieder angesaugt wird, was zur Folge hätte, dass die Atemluft zwar direkt am Gerät gefiltert wäre, etwas entfernt davon jedoch nicht mehr.

Durch den Vorfilter werden die UV-C Lampen und der Hauptfilter geschützt Hierdurch wird die Wartung erleichtert. Während der Vorfilter etwa jedes halbe Jahr gewechselt werden muß, genügt es, den Hauptfilter alle zwei Jahre auszutauschen. (*)
(*) im OP-Bereich kann as, bedingt durch den hohen Fusselgehalt des Mullbinden-Verbandmaterials, erfordertich sein, den Vorfilter alle drei Monate zu wechseln.

Dadurch, daß die Bakteien, Viren, Mikroorganismen erst abgetötet werden, bevor sie zu dem Hauptfilter gelangen, können Sie sich in diesem nicht vermehren bzw. Hindurch wachsen.

Die Lampenhalterung 30 ragt über die Trennebene 16 von unterem und oberen Rohrteil 15 / 16 hinaus. Die Enden 31 der Lampenhalterung 30 sind leicht abgeschrägt. Hierdurch wird ein leichtes Aufsetzen und eine Führung bei dem Zusammenbau der Rohrteile ermöglicht. Weiterhion befindet sich auch ein kleiner Teil der UV-C Lampen 22 oberhalb der Trennebene 16. Dies dient dazu, daß nach Abnahme des oberen Rohrteiles 15 die UV-C Lampen mit der Hand noch gut zugänglich angefaßt werden können für den Fall einer Erneuerung.

### Weitere vorteilhafte Ausgestaltung:

Der Ausströmkegel 3 nimmt in seinem Kegel eine Lampe 26 auf. Diese Lampe kann in weiterer Ausgestaltung eine Ringleuchte sein. Die Vorrichtung dient so als Stehlampe und da sie gegen die Decke strahlt, ist sie besonders als Deckenfluter geeignet

Mit 27 und 28 werden elektrische Schalter angezogen. Diese Schalter können auch als Regler ausgebildet sein, so daß die Helligkeit oder die Luftmenge eingestellt werden können.
Die Anlage ist für eine Luftmenge von 180 m³ pro Stunde absolut sauberer Luft gleichbleibender Qualität ausgelegt.
Die Anlage paßt auch in das Ambiente von Wohnzimmern.
Obwohl der Geräusonpegel so niedrig wie technisch sinnvoll machbar ist, er liegt bei etwa 60 db(A), kann der Benutzer, der z.B. an Heuschnupfen leidet, die Filterung der Atemluft absenken, die Menge reduzieren, wenn er Besserung verspürt und da mit den Geräuschpegel noch weiter verringern. Bei einem Luftdurchsatz von 60 m³ pro Stunde kann so der Geräuschpegel auf 46 db(A) gesenkt werden.

Um die Geräuschemmision gering zu halten, wird für die Regelung des Ventilators ein Stelltrafo vorgesehen.
Um neben der Geräuschemmision auch den Energieverbrauch niedrig zu halten, kann in weiterer Ausgestaltung der Erfindung der Ventilator mittels Frequenzwandlers gesteuert werden.
Die Innenfläche des Rohres 2, insbesondere der Bereich, der von den UV-C Lampen angestrahlt wird, kann in weiterer Ausgestaltung der Erfindung verspiegelt sein.
Hierdurch wird die Strahlung weniger schnell von der Wandung geschluckt. Sie durchläuft mehrfach den Raum. Die Entkeimung wird dadurch vorteilhaft beeinflußt.

Die Vorriontung kann sowohl aus Metall als auch aus Kunststoff Hergestellt werden. Wird die Vorrichtung aus Kunststoff gefertigt so schützt die Verspiegelung den Kunststoff vor den UV-C Strahlen.

Statt eine Schweißverbindung kann auch eine Klebeverbindung vorgenommen werden.

Bei Verwendung von Kunststoff kann die Innenfläche des Rohres mit einem elektrisch leitenden Lack versehen werden. Dieser leitet die durch die strömende Luft erzeugte statische Ladung zu einem Erdanschluß ab.

Desweitem können die Filter mit einem metallischen Netz umgeben sein, das geerdet ist
- 1: Gehäuse
- 2: Rohr
- 3: Ausströmkegel
- 4: Bodenplatte
- 5: Seitenwand
- 6: Seitenwand
- 7: Vorderwand
- 8: Rückwand
- 9: Öffnung
- 10: Öffnung
- 11: Blechzunge
- 12: Schraube
- 13: Öffnung
- 14: unterer Rohrteil
- 15: oberer Rohrteil
- 16: Trennebene
- 17: Kugel
- 18: Ventilator
- 19: Feinstaubfilter
- 20: Schlauchschelle
- 21: Gehäzuse
- 22: UV-C Lampe
- 23: Hauptfilter
- 24: Aktivkohlefilter
- 25: Bodenluft
- 26: Lampe
- 27: Schalter für Lampe
- 28: Schalter für Luft
- 29: Schraube
- 30: Lampenhalterung
- 31: Enden der Lampenhalterung

## Patentansprüche

1. Vorrichtung zum Reinstfiltern und Desinfizieren von Luft mit einem in oder an einem Gehäuse (1) angeordneten Feinstaubfilter (19), und einem Ventilator (18), sowie einem Rohr (2), in dem UV Lampen (22) zur Desinfektion der Luft, ein Hauptfilter (23) zur Feinstfilterung der Luft und ein Kohlefilter (24) angeordnet sind, **dadurch gekennzeichnet,** dass der Kohlefilter Aktivkohle umfasst, und dass ein Ausströmkegel (3) vorgesehen ist, der mit dem Ende des Rohrs einen Spalt bildet, und dass der Austritt zwischen 1,5 und 2, 5 m oberhalb einer Lufteintrittsöffnung (9) angeordne ist.

2. Vorrichtung nach Anspruch 1, **dadurch gekennzeichnet,** dass das Gehäuse (1) besteht aus:
a) einer Grundplatte (4)
b) zwei Seitenwänden (5, 6), die auf der Grundplatte (4) befestigt sind,
c) einer Vorderwand (7), die mit den Seitenwänden (5, 6) sowie der Grundplatte (4) verbunden ist, und
d) einer Rückwand (8).

3. Vorrichtung nach Anspruch 2, **dadurch gekennzeichnet,** dass in mindestens einer der Wände (5, 6, 7, 8) die Lufteintrittsöffnung (9) vorgesehen ist

4. Vorrichtung nach einein der Ansprüche 1 bis 3, **dadurch gekennzeichnet,** dass der Ventilator (8) mittels schallisolierender Mittel befestigt ist.

5. Vorrichtung nach einem der Ansprüche 1 bis 4, **dadurch gekennzeichnet,** dass das Rohr aus einem unteren Rohrteil (14) und einem oberen Rohrteil (15) besteht, und in dem unteren Rohrteil (14) die UV-Lampen (22) und in dem oberen Rohrteil (15) der Hauptfilter (23) und der Aktivkohlefilter (24) angeordnet sind.

6. Vorrichtung nach einem der Ansprüche 1 bis 5, **dadurch gekennzeichnet,** dass die UV-Lampen (22) UV-C Lampen sind.

7. Vorrichtung nach einem der Ansprüche 1 bis 6, **dadurch gekennzeichnet,** dass die Innenfläche des Rohres (2) teilweise verspiegelt ist.

8. Vorrichtung nach einem der Ansprüche 1 bis 6, **dadurch gekennzeichnet,** dass die Innenfläche des Rohres (2) mit einem elektrisch leitenden Lack versehen ist.

9. Vorrichtung nach einem der Ansprüche 1 bis 8, **dadurch gekennzeichnet,** dass mindestens ein Filter mit einem geerdeten metallischen Netz umgeben ist.

10. Vorrichtung nach einem der Ansprüche 1 bis 9, **dadurch gekennzeichnet,** dass der Ausströmkegel (3) auf mehreren auf der Stimfläche des Rohres (2) angeordneten Kugeln (17) befestigt ist.

11. Vorrichtung nach einem der Ansprüche 1 bis 10, **dadurch gekennzeichnet,** dass auf dem Ausströmkegel (3) eine Lampe (26) angeordnet ist.

12. Vorrichtung nach einem der Ansprüche 1 bis 11, **dadurch gekennzeichnet,** dass mittels Schalter (27, 28) die durchströmende Luftmenge regulierbar ist.

## Claims

1. Device for the sheer filtering and disinfection of air, with a fine dust filter (19) arranged in or on a housing (1), and a fan (18), and also a tube (2), in which UV lamps (22) for the disinfection of the air, a main filter (23) for particulate filtering of the air and a charcoal filter (24) are arranged, characterized in that the charcoal filter comprises active charcoal, and in that an outflow cone (3), which forms a gap with the end of the tube, is provided, and in that the outlet is arranged between 1.5 and 2.5 m above an air inlet opening (9).

2. Device according to Claim 1, characterized in that the housing (1) comprises:
a) a base plate (4)
b) two side walls (5 6), which are fastened on the base plate (4),
c) a front wall (7), which is connected to the side walls (5, 6) and also the base plate (4), and
d) a rear wall (8).

3. Device according to Claim 2, characterized in that the air inlet opening (9) is provided in at least one of the walls (5, 6, 7, 8).

4. Device according to one of Claims 1 to 3, characterized in that the fan (18) is fastened by means of sound-insulating means.

5. Device according to one of Claims 1 to 4, characterized in that the tube comprises a lower tube part (14) and an upper tube part (15), and the UV lamps (22) are arranged in the lower tube part (14) and the main filter (23) and the active charcoal filter (24) are arranged in the upper tube part (15).

6. Device according to one of Claims 1 to 5, characterized in that the UV lamps (22) are UV-C lamps.

7. Device according to one of Claims 1 to 6, characterized in that the inner surface of the tube (2) is partly mirrored.

8. Device according to one of Claims 1 to 6, characterized in that the inner surface of the tube (2) is provided with an electrically conducting lacquer.

9. Device according to one of Claims 1 to 8, characterized in that at least one filter is surrounded by an earthed metallic mesh.

10. Device according to one of Claims 1 to 9, characterized in that the outflow cone (3) is fastened on a plurality of balls (17) arranged on the end face of the tube (2).

11. Device according to one of Claims 1 to 10, characterized in that a lamp (26) is arranged on the outflow cone (3).

12. Device according to one of Claims 1 to 11, characterized in that the amount of air flowing through can be regulated by means of switches (27, 28).

## Revendications

1. Dispositif de filtrage ultra-pur et de désinfection de l'air avec un filtre fin (19) monté dans ou sur un coffret (1), et comportant un ventilateur (18) ainsi qu'un tube (2), dans lequel sont montées des lampes à rayons UV (22) pour désinfecter l'air, un filtre principal (23) de filtrage très pur de l'air et un filtre à charbon (24), caractérisé en ce que le filtre à charbon comprend du charbon actif, et en ce qu'un cône de sortie (3) est prévu, qui forme un interstice avec l'extrémité du tube et en ce que la sortie est située à entre 1,5 m et 2,5 m au-dessus d'une ouverture d'entrée d'air (9).

2. Dispositif selon la revendication 1, caractérisé en ce que le coffret (1) est constitué de :
a) une plaque de fond (4)
b) deux parois latérales (5, 6) fixées sur la plaque de fond (4),
c) une paroi avant (7) assemblée aux parois latérales (5, 6) ainsi qu'à la paroi de fond (4), et
d) une paroi arrière (8).

3. Dispositif selon la revendication 2, caractérisé en ce qu'une ouverture d'entrée d'air (9) est prévue dans au moins l'une des parois (5, 6, 7, 8).

4. Dispositif selon l'une des revendications 1 à 3, caractérisé en ce que le ventilateur (18) est fixé à l'aide d'un moyen isolant phonique.

5. Dispositif selon l'une des revendications 1 à 4, caractérisé en ce que le tube est constitué d'une partie de tube inférieure (14) et d'une partie de tube supérieure (15), et en ce que les lampes à rayons UV (22) sont montées dans la partie de tube inférieure (14) et le filtre principal (23) et le filtre au charbon actif (24) sont montés dans la partie de tube supérieure (15).

6. Dispositif selon l'une des revendications 1 à 5, caractérisé en ce que les lampes (22) sont des lampes à rayons UV-C.

7. Dispositif selon l'une des revendications 1 à 6, caractérisé en ce que la surface intérieure du tube (2) est partiellement revêtue d'une couche réfléchissante.

8. Dispositif selon l'une des revendications 1 à 6, caractérisé en ce que la surface intérieure du tube (2) est revêtue d'une peinture conductrice d'électricité.

9. Dispositif selon l'une des revendications 1 à 8, caractérisé en ce qu'au moins un filtre est entouré d'un filet métallique relié à la terre.

10. Dispositif selon l'une des revendications 1 à 9, caractérisé en ce que le cône de sortie (3) est fixé sur plusieurs billes (17) réparties sur la surface frontale du tube (2).

11. Dispositif selon l'une des revendications 1 à 10, caractérisé en ce qu'une lampe est montée sur le cône de sortie (3).

12. Dispositif selon l'une des revendications 1 à 11, caractérisé en ce qu'il est possible de régler le débit d'air au moyen de commutateurs (27, 28).
